# EUROPEAN PATENT SPECIFICATION

(11) **EP 1 372 161 B1**
(45) Date of publication and mention of the grant of the patent: **25.05.2005**
(21) Application number: 01909851.6
(22) Date of filing: 13.03.2001
(51) Int. Cl.: H01C 7/02, H01C 1/014, H01C 1/084, A61L 9/03, A01M 1/20

(54) **MULTI-USE HEATING DEVICE FOR THE EVAPORATION OF ACTIVE SUBSTANCES**
MEHRZWECKHEIZVORRICHTUNG ZUR VERDAMPFUNG AKTIVER SUBSTANZEN
DISPOSITIF CHAUFFANT A USAGES MULTIPLES PERMETTANT L'EVAPORATION DE SUBSTANCES ACTIVES

(43) Date of publication of application: 17.12.2003
(73) Proprietor: Zobele Espana, S.A., 08290 Cerdanyola del Vallés (ES)
(72) Inventor: BASAGANAS MILLAN, Jordi, E-08290-Cerdanyola Del Valles (ES); CASERTA Andrea, E-08290 Cerdanyola Del Valles (ES); GARCIA FABREGA Ruben, E-08290 Cerdonyola Del Valles (ES)
(74) Representative: Carpintero Lopez, Francisco
(86) International application number: PCT/ES2001/000094
(87) International publication number: WO 2002/073637

(56) References cited:
- EP-A1- 0 689 766
- WO-A1-00/06212
- ES-T3- 2 112 363
- US-A- 4 973 934
- US-A- 5 142 265

## Description

### PURPOSE OF THE INVENTION

This invention refers to a heater device for the evaporation of active substances, such as those used as insecticides or air-fresheners, of the type that use an electrical resistance, preferably of the PTC type, as means of heating. The heater has a multiuse character, that is that it is structured to be able to carry out the evaporation of products in liquid state, in tablets or in the form of gel, indistinctly, achieving an optimum performance independent of the nature of the product to be vaporized.

### BACKGROUND TO THE INVENTION

Electrically operated heater devices exist that produce the evaporation of liquids held in diffusers, in some cases, in other cases solids, in the form of tablets; and sometimes they even allow the use of tablets or liquids indistinctly, according to choice.

The devices presently known for the evaporation of those products are usually fabricated using a PTC type electrical resistance connected to a pair of metallic contacts. When activated, the electrical resistance heats a ceramic or metallic plate through which, and placed near a tablet diffuser, produces the evaporation of the corresponding substance. On the other hand, the heater device can have an opening in which a wick is placed, that is brought out to the exterior of a diffuser vessel holding a liquid product; so that the heating of that wick produces the evaporation of the liquid substance held in the vessel.

For that reason, a single heater device is not advisable, nor efficient, for carrying out the evaporation of products in tablet form and liquid form, indistinctly, since the optimum working temperature of the wick when liquid products are concerned doesn't coincide with that of a tablet, when solid products are concerned. Therefore two types of heater devices are usually sold, one specific for liquids and another, also specific, for solids or tablets.

In attempting to prevent this problem this applicant is holder of the Spanish Invention Patent, with Application number 9801592, that describes a multiuse heater device in which the electrical resistance is complemented with an aluminium plate acting as a radiator, that is adequately held inside the corresponding body. Both the body and the aluminium plate having an opening through which a wick may be passed emerging through the mouth of a vessel that holds a liquid product; at the same time that a frame type separator element is placed on the body, with means of attachment to the body, forming a support surface for a tablet that can thus be separated to a greater or lesser extent from the heating plate by means of the separating element, so that the tablet always has adequate heat to achieve its evaporation with maximum efficiency.

This solution is structurally complex since two radiators are needed, one for heating the tablet and another for heating the wick, since exclusively using a radiator for the tablet, the heat transfer to the wick could be insufficient.

However, the aluminium plate that acts as a radiator is kept physically independent of the PTC resistance by an insulating film that hinders the transfer of heat from the resistance to the radiator or aluminium plate.

Furthermore, the connection contacts of the PTC resistance as well as being independent from each other, are also independent from the radiator, which multiplies the number of parts and complicates the structure of the device.

### DESCRIPTION OF THE INVENTION

The heater device that the invention proposes is defined by the features of claim 1. It fully and satisfactorily resolves the forementioned difficulties, in each and every of the different aspects commented upon, forming a solution of maximum structural simplicity and optimum operation, with perfect homogeneity in the extension of heat to a pellet, on all its receptive surfaces, and also with an notable provision of heat to a wick, in the case that the product is liquid coming from a container vessel, also having a uniform distribution of the heat to all the periphery of the wick.

For this purpose and more specifically the heater device that is proposed is fabricated starting with a plastic body which has two parts, a base and a cover, properly interconnectable and securable by any conventional means. The forementioned base is fitted with a circular opening extended into a cylindrical neck, for access of the wick in the case of coupling of the device to a vessel holding the product in liquid phase. This neck is coaxial with the other that exists in the cover, encircling the first and of greater diameter, to define between them a ring-shaped throat in which two aluminium plates are placed, curved and opposed, acting as a radiator, so that together they mainly grip the neck of the base through which the wick has to pass; these curved plates being a single-piece extension of other flat plates, from which they are obtained by die pressing,designed to adapt to the bottom of each of the two halves of the base of the body, impacting mainly on its surface,and designed for heating in the case of a pellet or solid product.

In accordance with another of the characteristics of the invention the two flat plates have orthogonally bent wings, also flat, that in the assembly are parallel to each other and sufficiently distanced to allow the placing between them of the PTC resistance, together with a spring that assures an optimum connection, so that the aluminium pieces not only form the thermal radiator, both for products in pellet form and for liquid products, but furthermore it forms part of the PTC resistance supply circuit, specifically forming the connection terminals between this resistance and the electrical supply cables of the device.

Obviously both the base of the heater and the supplementary cover will be duly configured for, after their assembly, duly immobilizing the different functional components of the device in their interior.

The bottom of the base acts externally as a support surface for the pellet of solid product, so that the degree of heating of the latter will be a function of the thickness of the wall of the bottom of the forementioned base piece of the heater body.

### DESCRIPTION OF THE DRAWINGS

To supplement this description and with the aim of leading to a better understanding of the characteristics of the invention, in accordance with a preferred example of its practical embodiment, as an integral part of this description it is accompanied by a set of drawings where in an illustrative and non-limiting way, the following have been represented:

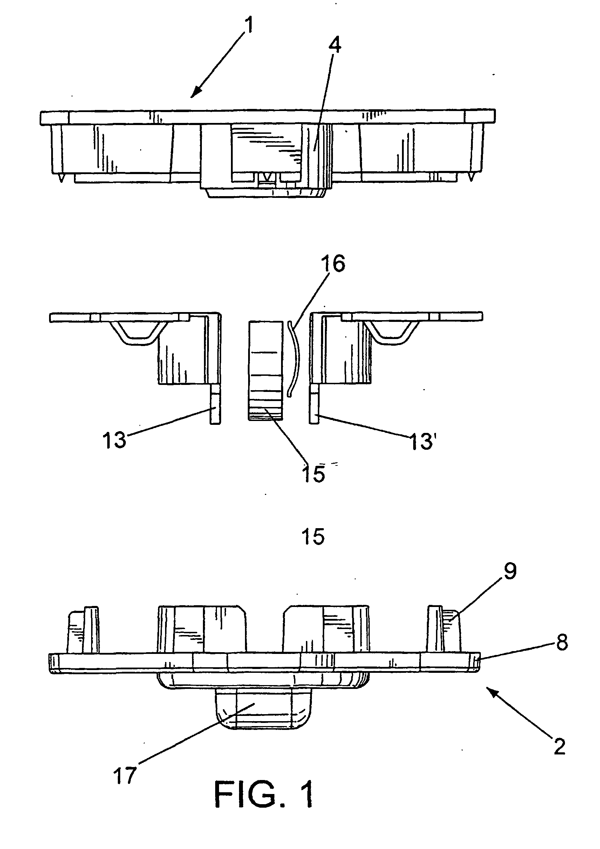

Figure 1.- Shows a side elevation and cut-away of a multiuse heating device for the evaporation of active substances, carried out in accordance with the aim of this invention.

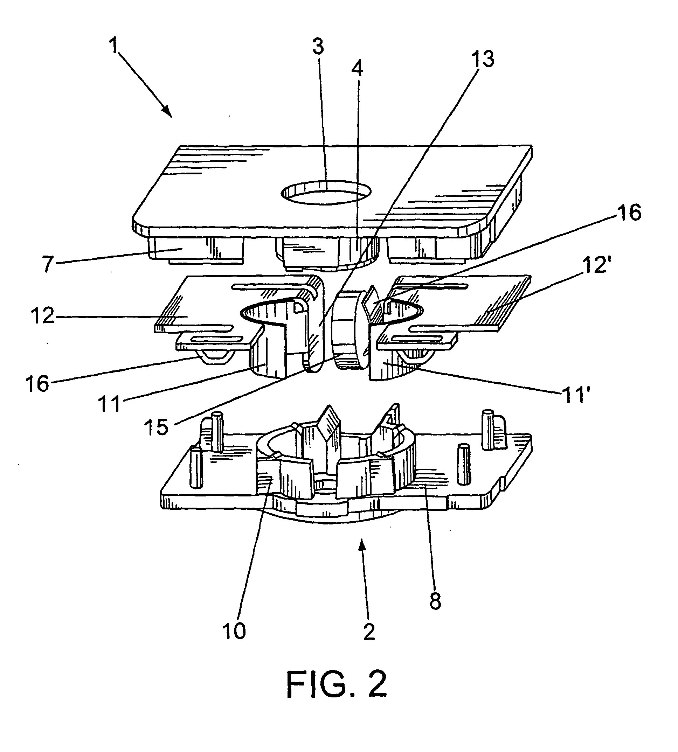

Figure 2.- Shows the same cut-away as the previous figure, now with a perspective view.

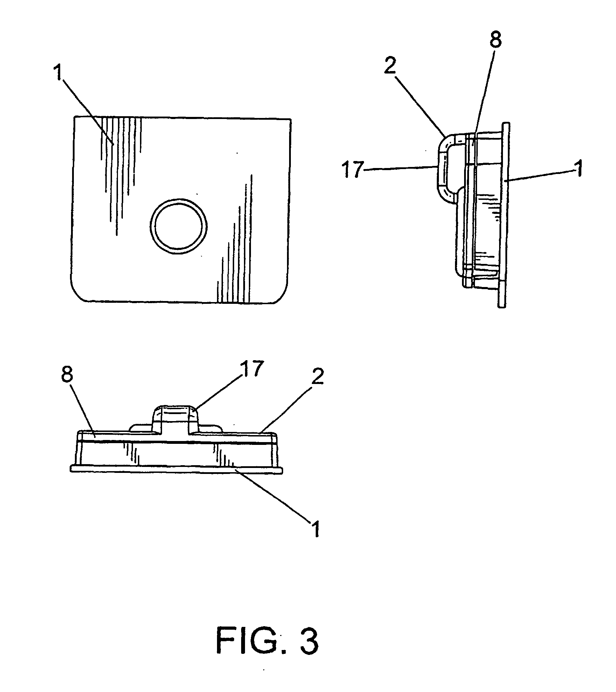

Figure 3. - Shows a plan view and two 90° profiles of the assembly of the previous figures, duly mounted.
Figure 4.- Finally shows in perspective view, the device of figure 3 duly attached to a vessel that supplies liquid product.

### PREFERABLE EMBODIMENT OF THE INVENTION

In view of the figures depicted it can be observed how the multiuse heater device that the invention proposes is fabricated starting from a body, which has a base (1) and a cover (2), a base (1) that forms a kind of approximately prismatic-rectangular bowl, with a perfectly flat bottom wall (1 ), fitted with a circular opening (3) extended in a neck (4), also cylindrical, of adequate dimensions to allow the wick (5), from a vessel (6) holding the product in liquid state, to be relatively tightly passed through it, when it is considered appropriate, while the same external surface of the bottom (1 ) forms the flat support base for a tablet, not shown in the drawings, when the use of a solid phase product is intended.

As well as the neck (4) rising from its bottom wall, the base (1 ) incorporates a peripheral partition (7), partially open, intended to receive, at its free edge, an essentially flat plate (8), forming the cover (2) and fitted with internal separators (9), as well as a cylindrical neck (10), also lateral and partially open, of greater diameter than the neck (4) of the base (1 ), intended to receive this latter in its cavity and to form with it a ring-shaped housing for a pair of curved partitions (11-11'), obtained by die pressing from two aluminium plates (12-12'), flat and in the same plane, designed to be seated on the bottom of the base (1 ), specifically leaving free this latter's neck (4) the said partitions (11) being placed in correspondence with this latter; at the same time that these aluminium plates (12-12') respectively incorporate fins (13), orthogonally bent in the same direction as the partitions (11 ), flat and parallel, destined to act as terminals between which the PTC electrical resistance (15) and a small spring (16) that assures its good electrical contact with the fins (13), are established, which through the same plates (12-12'), join to the connection terminals (16) for the respective electrical supply cables, not shown.

The dimensions of the PTC resistance (15), specifically its diameter, obliges an over-dimensioning of the body, to said effect the cover (2) incorporates a closed protuberance (17) that partially houses in its interior both the PTC resistance (15) and the extremity of the connection fins (13).

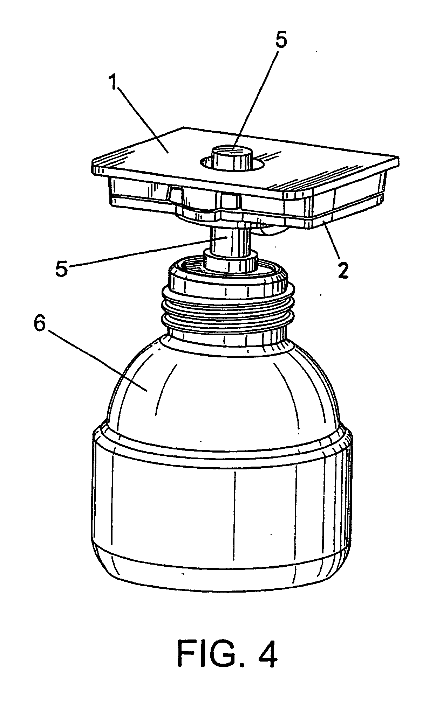

Thus it is achieved that the device can be applied to the wick (5) of the vessel (6) holding a liquid product, as shown in figure 4, where the optimum heating of the forementioned wick (5) is assured by the curved partitions (11 ) that mostly surround this wick (5) impinging on a broad sector of it. The device can similarly be used for products in solid state, in which case the opening or cylindrical housing (3) for the wick is inoperative, the pellet (not shown) being placed in position, on the flat surface of the base (1) of the heater; a surface that, in turn, is mostly impinged on by the flat internal aluminium plates (12-12') acting as a radiator, with an optimum and homogeneous thermal transfer.

## Claims

1. Multiuse heater device for the evaporation of active substances, being fabricated starting with a body in which an electrical resistance (15), PTC type, is placed, with its corresponding electrical supply connectors; an opening (3) being made in the body for passing a wick (5) from a vessel (6) holding the substance, when this is in liquid phase; a flat surface being defined in the body for positioning a pellet, when it is a substance in solid state; The device being of the type that has in its interior a radiator that allows the dissipation of the heat generated by the resistance (15); the device being **characterized by** said radiator being formed of two symmetrical aluminium plates (12-12'), in each one of which a main flat section is defined, these sections to be placed in the same plane, and each of them being die pressed forming a semi ― cylindrical curved partition (11-11'), and a flat fin (13-13'); while in the base (1 ) and cover (2) of the body twin coaxial necks (4) and (10) are established, forming between them a ring-shaped fluting in which the curved partitions (11-11') are established, to surround, in its case, the wick (5) supplying the product from the vessel (6), while the fins (13-13') adopt a parallel layout and receive between them the PTC resistance (15) and a metal strip or spring (16).

2. Multiuse heater device for the evaporation of active substances, according to claim 1, **characterised in that** the plates (12-12') forming the thermal dissipation radiator also act as electrical connectors for the PTC resistance (15), the spring (16) assuring the electrical contact of this resistance (15) with the fins (13-13'), opposite which and above the plates (12-12') the terminals (16) for connection of the respective electrical supply cables are set up.

3. Multiuse heater device for the evaporation of active substances, according to any of the previous claims, **characterised in that** the base (1) incorporates a peripheral partition (7), partially open, for holding in its interior the two metallic plates (12-12') that act as a radiator, this container being closed by means of the cover (2) fitted with separators (9) that participate in the stabilization of the radiator plate (12-12'), the aforementioned cover (2) having a protuberance (17) for housing the projecting section of the PTC resistance (15) and the connection arms (13-13').

4. Multiuse heater device for the evaporation of active substances, according to any of the previous claims, **characterised in that** the thickness of the wall of bottom of the base (1), on which the pellet must be supported, is variable according to the anticipated level of heat transfer to this pellet, in turn according to its characteristics.

## Patentansprüche

1. Mehrzweckerhitzungsvorrichtung zur Verdampfung von aktiven Substanzen, die aus einem Körper hergestellt wird, in dem ein elektrischer Widerstand (15) von der PTC-Art angebracht wird mit den entsprechenden Anschlüssen für die elektrische Versorgung; eine Öffnung (3), die in dem Körper gemacht wird, um einen Docht (5) hindurchzuführen von einem Behälter (6), der die Substanz aufnimmt, wenn sich diese in flüssigem Zustand befindet; eine flache Oberfläche, die in dem Körper ausgebildet ist, um einen Pressling aufzunehmen, wenn sich die Substanz im festen Zustand befindet; die Vorrichtung ist von der Art, die in ihrem Inneren über einen Heizköper verfügt, der die Verbreitung der vom Widerstand (15) erzeugten Wärme erlaubt; wobei die Vorrichtung **dadurch gekennzeichnet ist, dass** der besagte Heizkörper aus zwei symmetrischen Aluminiumplatten (12-12') besteht, in denen jeweils ein flacher Hauptabschnitt ausgebildet ist, die in der selben Ebene angeordnet werden und die beide druckgegossen werden, um einen halbzylindrisch gebogenen Teilbereich (11-11') zu bilden und eine flache Rippe (13-13'), während an der Basis (1) und dem Deckel (2) des Körpers koaxiale Doppelhälse (4) und (10) ausgebildet sind, die zwischen ihnen eine ringförmige Rinne bilden, in der die gebogenen Teilbereiche (11-11') festgelegt werden, um in diesem Fall den Docht (5) einzuschliessen und das Produkt von dem Behälter (6) zuzuführen, während die Rippen (13-13') eine parallele Anordnung aufweisen und zwischen sich den PTC-Widerstand (15) aufnehmen und einen Metallstreifen oder -feder (16).

2. Mehrzweckerhitzungsvorrichtung zur Verdampfung von aktiven Substanzen gemäss Anspruch 1, **dadurch gekennzeichnet, dass** die Platten (12-12'), die den Heizkörper zur thermischen Verbreitung bilden, auch als elektrische Anschlüsse für den PTC-Widerstand (15) funktionieren, wobei die Feder (16) den elektrischen Kontakt dieses Widerstands (15) mit den Rippen (13-13') gewährleistet, denen gegenüber und über den Platten (12-12') die Endstücke (16) für das jeweilige Stromversorgungskabel angebracht werden.

3. Mehrzweckerhitzungsvorrichtung zur Verdampfung von aktiven Substanzen gemäss einem der vorangegangenen Ansprüche, **dadurch gekennzeichnet, dass** die Basis (1) einen peripheren Teilbereich (7) umfasst, der teils offen ist, um in seinem Inneren zwei Metallplatten (12-12') aufzunehmen, die als Heizkörper funktionieren, wobei dieser Behälter durch einen Deckel (2) geschlossen wird, der mit Begrenzungen (9) ausgestattet ist, die zu der Stabilisierung der Heizkörperplatte (12-12') beitragen, wobei der vorgenannte Deckel (2) über einen Vorsprung (17) verfügt, um den vorspringenden Abschnitt des PTC-Widerstands (15) aufzunehmen und die Verbindungsarme (13-13').

4. Mehrzweckerhitzungsvorrichtung zur Verdampfung von aktiven Substanzen gemäss einem der vorangegangenen Ansprüche, **dadurch gekennzeichnet, dass** die Wandstärke des Bodens der Basis (1), auf der der Pressling zu liegen kommt, veränderbar ist in Abhängigkeit von dem erwarteten Niveau der Hitzeübertragung auf diesen Pressling wiederum in Abhängigkeit seiner Eigenschaften.

## Revendications

1. Dispositif réchauffeur multifonctions pour l'évaporation de substances actives, fabriqué à partir d'un corps dans lequel est mise en place une résistance électrique (15) de type PTC avec ses connecteurs d'alimentation électriques correspondants ; une ouverture (3) étant aménagée dans le corps pour passer une mèche (5) depuis un récipient (6) contenant la substance, lorsque celle-ci est en phase liquide ; une surface plate étant définie dans le corps pour positionner une pastille, lorsque c'est une substance à l'état solide ; le dispositif étant du type ayant en son sein un radiateur permettant la dissipation de la chaleur générée par la résistance (15) ; le dispositif étant **caractérisé par** ledit radiateur formé par deux plaques symétriques en aluminium (12-12'), dans chacune desquelles est défini une section plate principale, les sections étant destinées à être mises en place sur le même plan, et chacune desquelles est matricée à la presse en formant une cloison courbée semi-cylindrique (11-11'), et une ailette plane (13-13') ; tandis que sur la base (1) et le couvercle (2) du corps sont établis des collets coaxiaux jumeaux (4) et (10), en formant entre eux une cannelure annulaire dans laquelle sont établies les cloisons courbées (11-11'), pour entourer, le cas échéant, la mèche (5) fournissant le produit à partir du récipient (6) tandis que les ailettes (13-13') adoptent une disposition parallèle et reçoivent entre elles la résistance de type PTC (15) et une bande ou ressort (16).

2. Dispositif réchauffeur multifonctions pour l'évaporation de substances actives, selon la revendication 1, **caractérisé en ce que** les plaques (12-12') formant le radiateur de dissipation thermique agissent aussi comme des connecteurs électriques pour la résistance de type PTC (15), le ressort (16) assurant le contact électrique de la résistance (15) avec les ailettes (13-13'), en regard desquelles et par-dessus les plaques (12-12') sont disposées les bornes (16) pour la connexion des câbles d'alimentation électrique respectifs.

3. Dispositif réchauffeur multifonctions pour l'évaporation de substances actives, selon l'une quelconques des revendications antérieures, **caractérisé en ce que** la base (1) incorpore une cloison périphérique (7) partiellement ouverte, pour contenir en son sein les deux plaques métalliques (12-12') qui agissent comme un radiateur, ce conteneur étant fermé au moyen du couvercle (2) pourvu de séparateurs (9) qui participe à la stabilisation de la plaque de radiateur (12-12'), ledit couvercle (2) ayant une protubérance (17) pour loger la section saillante de la résistance de type PTC (15) et les bras de connexion (13-13').

4. Dispositif réchauffeur multifonctions pour l'évaporation de substances actives, selon l'une quelconque des revendications antérieures, **caractérisé en ce que** l'épaisseur de la paroi de fond de la base (1), sur laquelle doit être supportée la pastille, est variable selon le niveau anticipé de transfert de chaleur à cette pastille, en accord à son tour à ses caractéristiques.
